# DEMANDE DE BREVET EUROPEEN

(11) **EP 0 707 832 A1**
(43) Date de publication de la demande: **24.04.1996**
(21) Numéro de dépôt: 95420276.8
(22) Date de dépôt: 10.10.1995
(51) Int. Cl.: A61B 17/60

(54) **Fixateur externe**

(30) Priorité: 12.10.1994 FR 9412495
(71) Demandeur: Hardy, Jean-Marie, F-19600 Larches (FR)
(72) Inventeur: Hardy, Jean-Marie, F-19600 Larches (FR)
(74) Mandataire: Thivillier, Patrick

(57) **Abrégé**

Fixateur externe applicable en orthopédie et un traumalogie, comprenant des barres (1) en matériau composite, chaque barre (1) recevant, avec capacité de réglage en translation et de blocage en position, des organes indépendants d'ancrage dans l'os, constitués par un collier (2) recevant une broche (3) conformée pour être visée dans l'os, ladite broche (3) étant bloquée sur le collier en combinaison avec un moyen rapporté (4). Le fixateur est caractérisé en ce que le collier (2) enserre la demi circonférence d'une barre (1) avec capacité de déplacement le long de la barre et présente deux branches (2a) et (2b) recevant la broche (3) bloquée par le moyen (4) constitué par une clavette apte à être inserrée entre les branches (2a) et (2b), en appui sur les génératrices de la barre (1), au moyen d'une face (4a) de forme complémentaire, ladite broche (3) traversant les branches du collier, dans un plan perpendiculaire aux génératrices de la barre, pour être positionnée au dessus d'une face inclinée (4b), tandis qu'un organe de serrage (5) assure le déplacement de ladite clevatte, à l'encontre de la broche en vue de sa fixation.

## Description

On connaît différents types de fixateurs externes pour des interventions orthopédiques, afin de procéder à des ostéosynthèses d'un membre quelconque. Ces fixateurs ont pour but d'assurer la réduction et la contention d'une fracture.

Généralement, un fixateur comprend des mâchoires montées en combinaison avec un corps. Chaque mâchoire recoit des broches d'ancrage pour être fixée de part et d'autre du foyer de fracture. La plupart des fixateurs connus ne sont pas modulaires, de sorte qu'il n'est pas possible de les adapter à l'évolution des cas pathologiques à traiter. Il en résulte une application figée lors de la première intervention. De même, il n'est pas possible d'adapter le fixateur au fur et à mesure du traitement et de l'évolution de la fracture considérée.

Le fixateur décrit dans la demande de brevet FR 2 683 446, dont le demandeur de la présente est également titulaire, remédie à ces inconvénients et permet d'assurer l'immobilisation d'une fracture avec conservation des mouvements d'articulation, ainsi que l'immobilisation en position. Pour l'essentiel, cette demande de brevet décrit un fixateur externe entièrement modulable, et qui comprend des éléments faisant office de mâchoires recevant les broches d'ancrage, pour être fixées dans une position déterminée, de part et d'autre du foyer de fracture. Les mâchoires sont aptes à recevoir, après fixation, au moins un corps avec capacité d'orientation angulaire contrôlée et de blocage dans cette position, au moyen de tiges d'accouplement.

Compte-tenu de son caractère modulable, un tel fixateur s'avère particulièrement efficace.

A partir de cette conception et de ce caractère modulable, l'invention se propose de réaliser un fixateur dont les éléments constitutifs permettent de réduire de manière importante son poids. En outre, la conception de ce fixateur permet son application, pour répondre à tous les besoins de la traumatologie et de l'orthopédie. En clair, le problème que se propose de résoudre l'invention, est de réaliser un fixateur externe léger, modulaire et évolutif, simple à utiliser, d'un coût de revient réduit, et en partie radio-transparent.

Un autre problème important que se propose de résoudre l'invention est d'avoir un système de broches unitaires, afin que chaque broche soit fixée directement sur une barre, par un système qui lui est propre.

Pour résoudre un tel problème, il a été conçu et mis au point un fixateur du type de ceux comprenant de manière connue des barres en matériau composite, chaque barre recevant, avec capacité de réglage en translation et de blocage en position, des organes indépendants d'ancrage dans l'os, constitués par un collier recevant une broche conformée pour être vissée dans l'os, ladite broche étant bloquée sur le collier en combinaison avec un moyen rapporté.

Selon l'invention, compte tenu des problèmes posés à résoudre, le fixateur est remarquable en ce que le collier enserre la demi circonférence d'une barre avec capacité de déplacement le long de la barre et présente deux branches et recevant la broche bloquée par le moyen constitué par une clavette apte à être inserrée entre les branches , en appui sur les génératrices de la barre, au moyen d'une face de forme complémentaire, ladite broche traversant les branches du collier, dans un plan perpendiculaire aux génératrices de la barre, pour être positionnée au dessus d'une face inclinée de la clavette, tandis qu'un organe de serrage assure le déplacement de ladite clavette, à l'encontre de la broche en vue de sa fixation.

Un autre problème que se propose de résoudre l'invention, est de pouvoir réunir et bloquer plusieurs barres selon différentes angulations possibles.

Pour résoudre un tel problème, les extrémités des barres sont aptes à être accouplées à différents types d'éléments conformés pour faire varier leur longueur et/ou orientation angulaire.

Plus particulièrement, le problème posé d'assurer l'accouplement des barres est résolu en ce que les éléments d'accouplement des barres sont constitués par un embout délimitant un chambrage interne apte à recevoir l'extrémité de la barre et présentant, à son extrémité, une portée conique mâle avec capacité de déformation élastique, à l'opposé de la portée conique, l'embout reçoit une douille taraudée présentant une portée interne tronconique femelle complémentaire à la précédente, pour assurer le blocage de la barre sous l'effet de déformation de la portée conique mâle résultant du vissage de ladite douille.

Le chambrage interne est rainuré pour augmenter l'accrochage sur la barre.

Pour résoudre le problème posé d'assurer le montage des barres selon différentes positions angulaires, les éléments d'articulation comprennent un embout apte à recevoir la barre, ledit embout présentant une rotule sphérique dont le siège est une douille conformée pour être vissée sur un corps équipé intérieurement d'une tête d'appui assujetti à une clavette présentant une pente pour faire plaquer, ladite tête contre la rotule en position vissée de son siège et dans la partie angulaire souhaitée de l'embout.

Avantageusement, les éléments d'articulation et d'accouplement sont montés en bout d'un corps.

Pour résoudre le problème posé d'assurer l'allongement d'un membre, le corps présente une tige d'allongement filetée vissée dans le corps et déplaçable par un écrou bloqué en translation dans un agencement du corps, un contre écrou assurant le blocage en translation de la tige.

L'invention est exposée, ci-après plus en détail à l'aide des dessins annexés, dans lesquels :

La figure 1 est une vue montrant les principaux éléments de base du fixateur.

La figure 2 est une vue en perspective montrant la mise en place d'une barre en combinaison avec les organes de serrage.

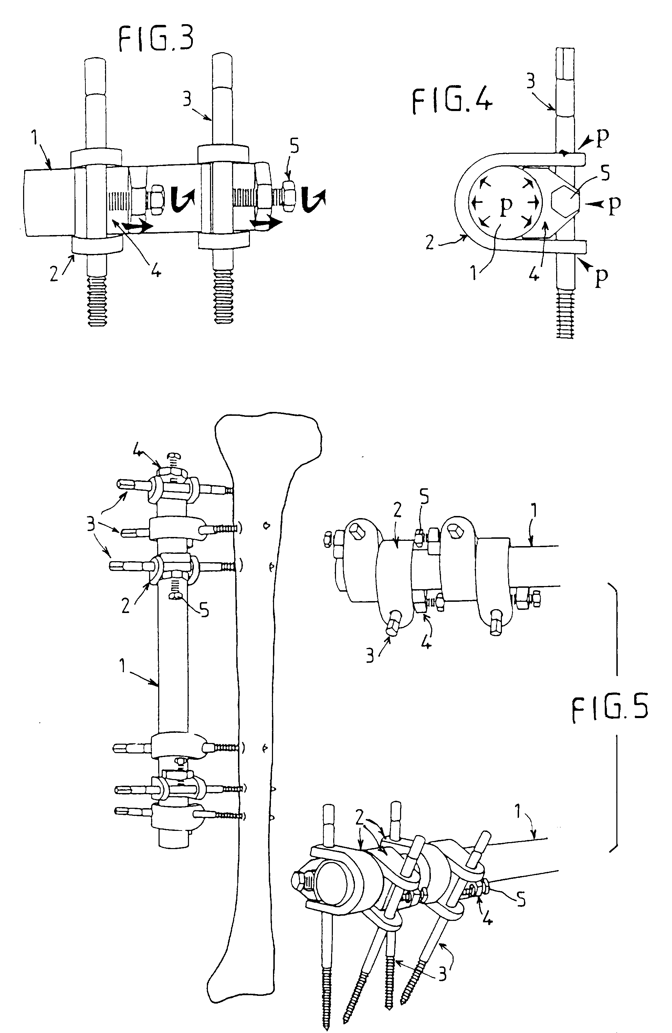

La figure 3 est une vue de dessus d'une barre équipée des organes de serrage.

La figure 4 est une vue de dessus correspondant à la figure 3,

La figure 5 est une vue en perspective d'un montage tibial du type monobarre, les broches étant disposées en forme de V sur la barre.

La figure 6 est une vue montrant l'utilisation de colliers doubles pour réaliser la jonction des deux barres d'un montage tibial en V.

La figure 7 est une vue en perspective montrant l'accouplement de deux barres par une troisième barre faisant office de poignée.

Les figures 8, 9 et 10, 11 et 12,13 sont respectivement des vues de face et de profil montrant différents types de montage dans le selon la stabilité primaire usinée, dans un montage monobarre.

Les figures 14, 15 et 16, 17 et 18, 19 sont des vues montrant des exemples de montage possibles selon la stabilité primaire désirée dans un montage bibarre.

Les figures 20, 21 et 22 sont des vues de dessus correspondant respectivement aux figures 14, 16 et 18.

Les figures 23, 24 et 25 sont des vues en coupe montrant le montage des barres en combinaison avec les éléments d'articulation.

La figure 26 est une vue en perspective d'une forme de réalisation d'un élément d'accouplement.

La figure 27 est une vue en perspective montrant le montage d'une barre avec un élément d'accouplement.

La figure 28 est une vue montrant une forme de réalisation du corps.

La figure 29 montre le corps équipé d'une tige d'allongement.

La figure 30 est une vue en perspective montrant un détail de réglage de l'allongement du corps.

La figure 31 est une vue en perspective montrant un allongement métaphysaire inférieur du fémur.

La figure 32 est une vue en perspective montrant les éléments de renvoi d'angle.

La figure 33 est une vue en perspective montrant le renvoi d'angle placé entre un allongeur et un corps.

La figure 34 est une vue en perspective dans le cas d'une ligamentotaxis de la cheville montée en combinaison avec un renvoi d'angle.

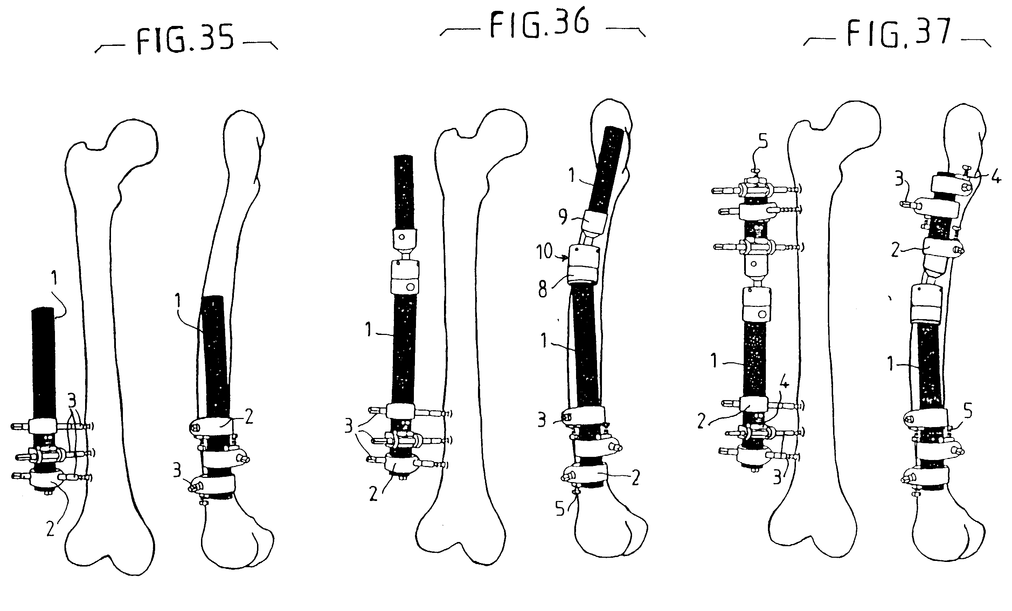

Les figures 35, 36 et 37 sont des vues en perspective montrant les principales phases de montage d'une barre, en combinaison avec les éléments d'articulation.

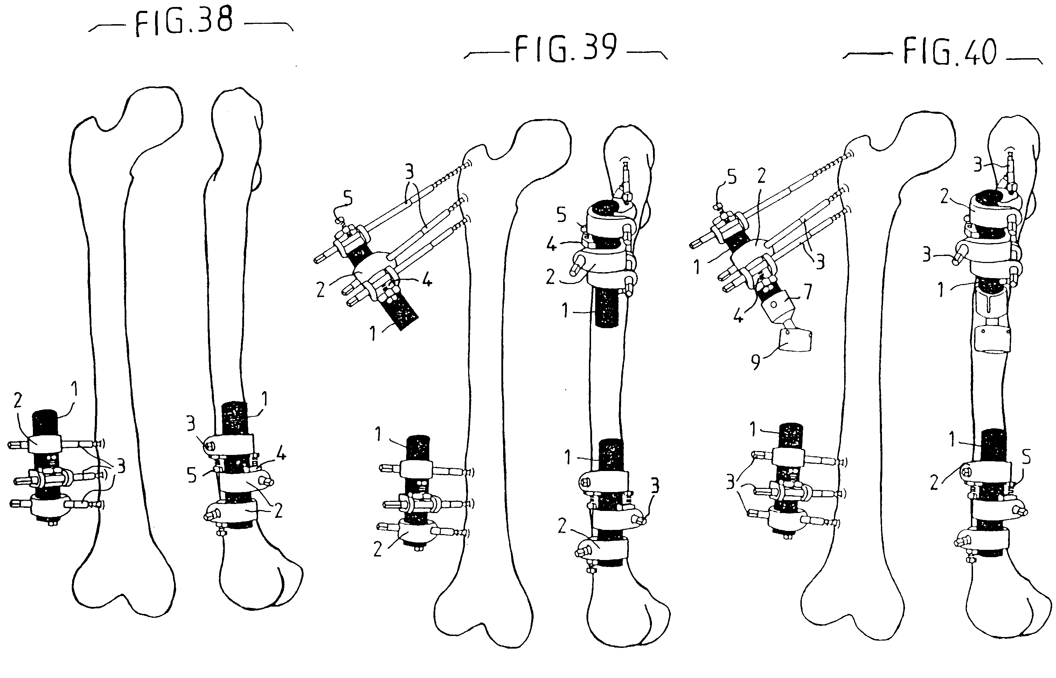

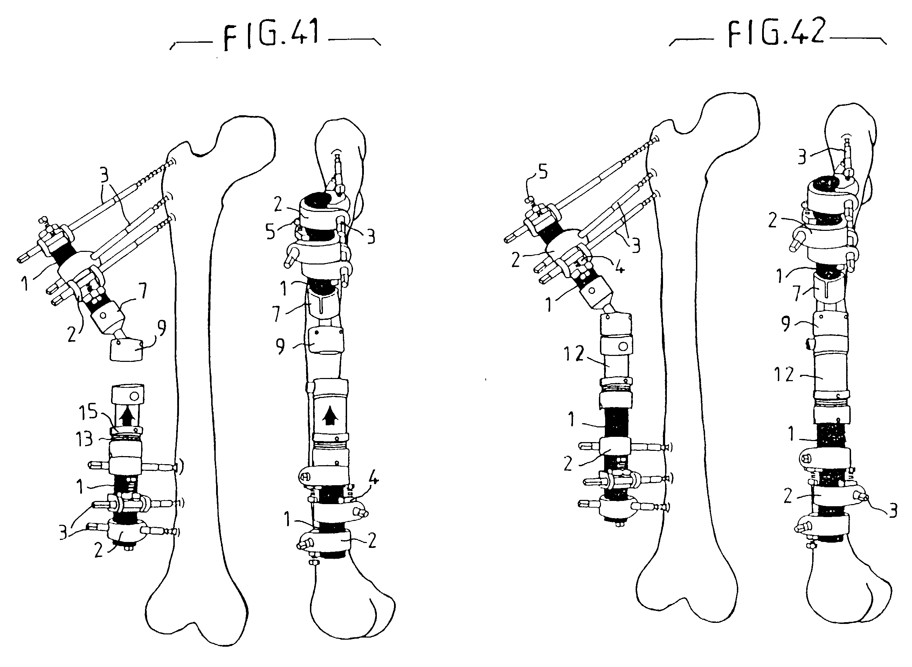

Les figures 38, 39, 40, 41 et 42 sont des vues montrant d'autres phases de montage d'un autre exemple de réalisation.

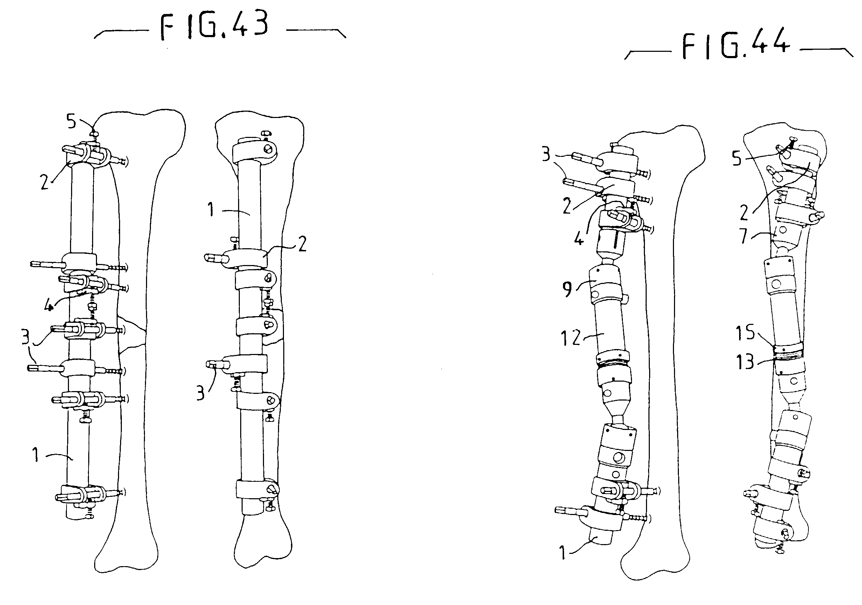

La figure 43 est une vue montrant un exemple de montage du type monobarre simple.

La figure 44 est une vue montrant un exemple de montage du type monobarre composite

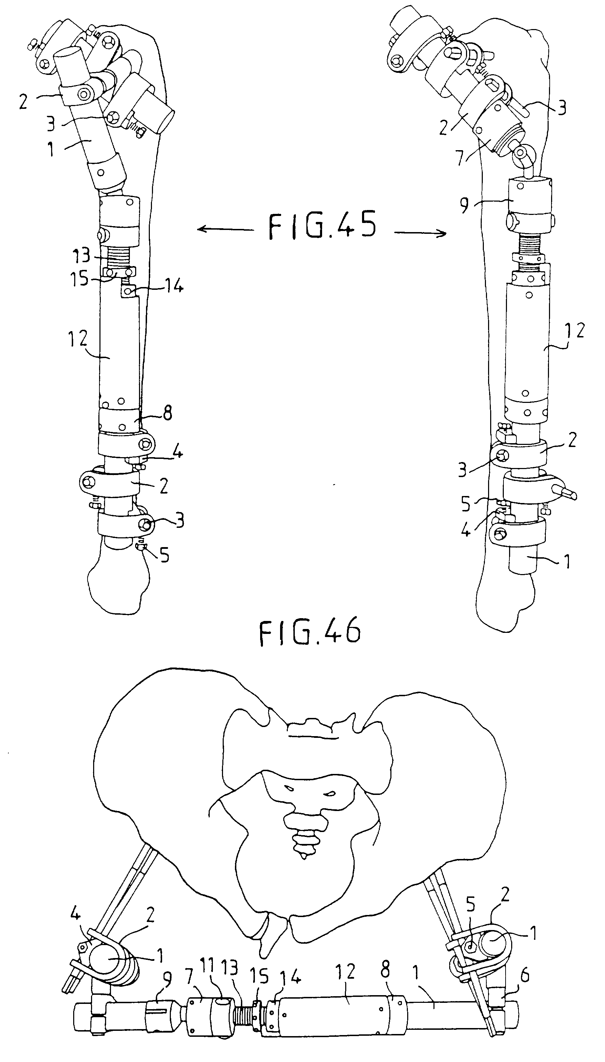

La figure 45 est une vue montrant un exemple de montage du fixateur dans le cas d'une fracture d'extrémité supérieure du tibia.

La figure 46 est une vue montrant un exemple de montage du fixateur dans le cas d'une fracture du bassin.

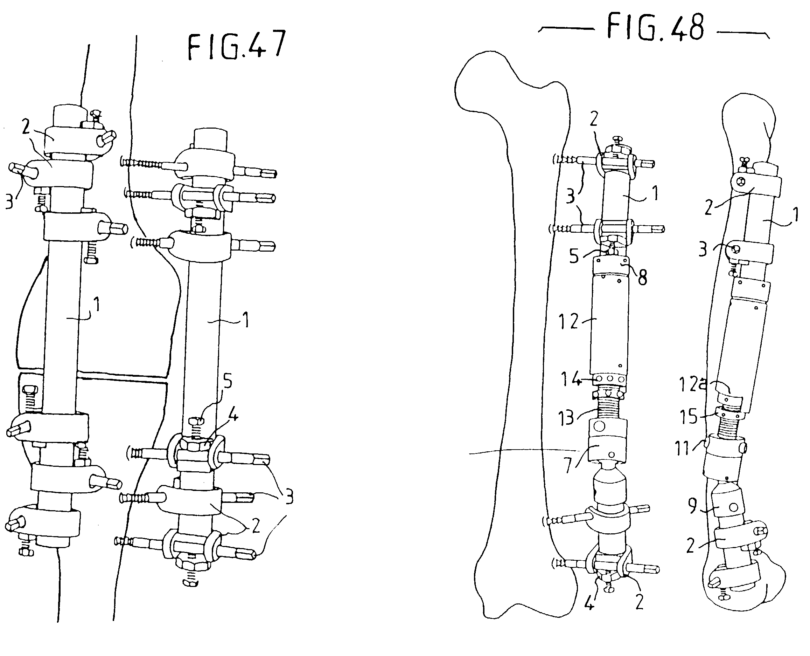

La figure 47 est une vue montrant un exemple de montage du fixateur dans le cas d'une arthrodèse du genou.

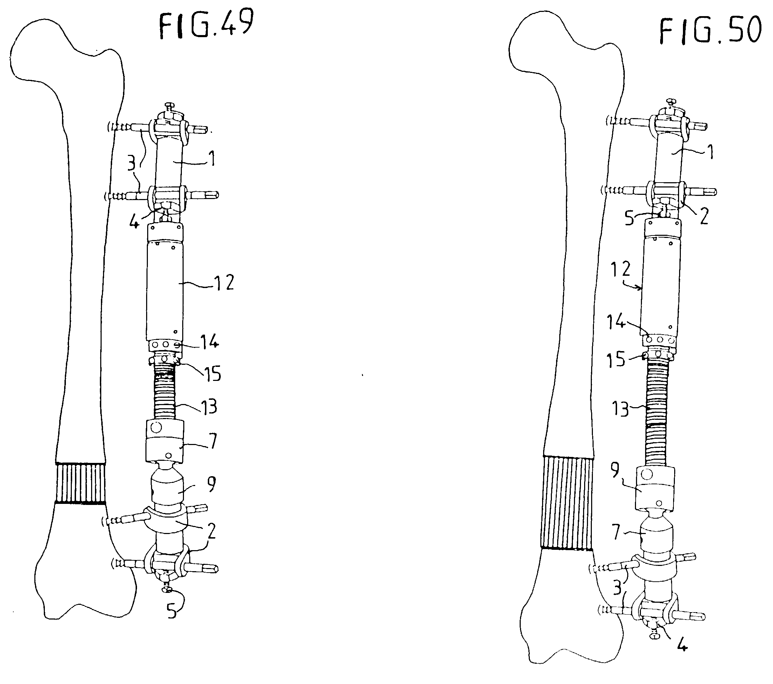

Les figures 48, 49 et 50 sont des vues en perspective montrant l'allongement métaphysaire inférieur du fémur, au moyen du fixateur.

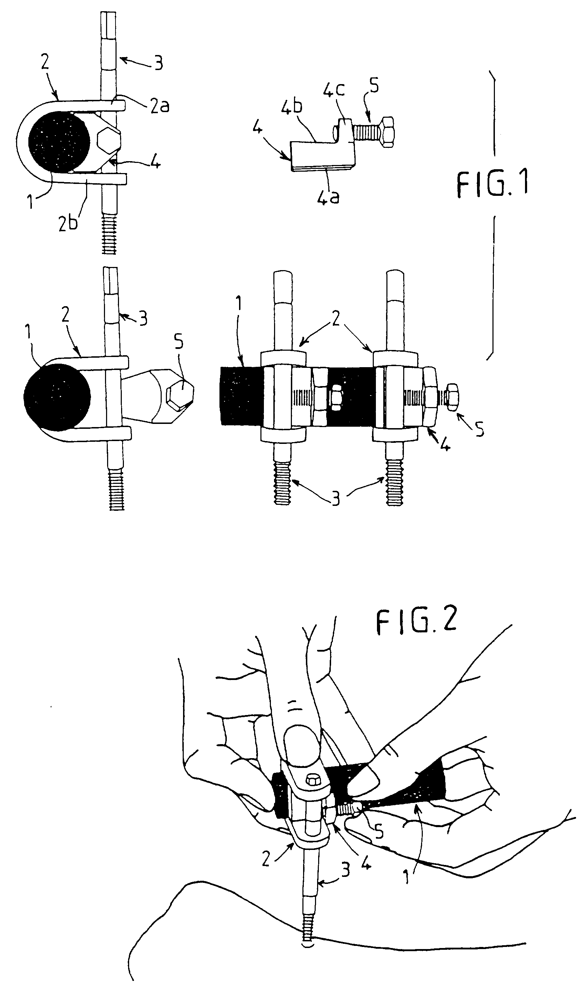

Comme le montre la figure 1, le fixateur comprend, à la base, des barres (1) en matériau composite, notamment en carbonne, apte à être coupées à la longueur souhaitée. Ces barres (1), qui sont radiotransparentes, coopèrent avec différentes pièces spécifiques, pour obtenir différents montages possibles, en fonction du cas pathologique à traiter. Les barres de carbone (1) peuvent être sectionnées facilement en per ou pré-opératoire.

Comme il sera indiqué dans la suite de la description, le montage des barres en carbone (1), en fonction des éléments spécifiques auxquels elles sont accouplées, permet de réaliser une pluralité de configurations.

Selon une caractéristique à la base de l'invention, les barres de carbone (1) reçoivent, avec capacité de réglage en translation et de blocage en position, des organes indépendants d'ancrage dans l'os. Ces organes sont constitués par un collier (2) aptes à enserrer la demi-circonférence d'une barre (1), avec capacité de déplacement le long de la barre. Les branches (2a) et (2b) du collier reçoivent une broche d'ancrage (3), de tout type connu et approprié, conformée pour être vissée dans l'os. La broche (3) est bloquée sur le collier (2), en combinaison avec un moyen rapporté (4). Plus particulièrement, ce moyen (4) est constitué par une clavette apte à être inserrée entre les branches (2a) et (2b) du collier (2), en appui sur les génératrices de la barre (1), au moyen d'une face (4a) de forme complémentaire.

La broche (3) traverse les branches (2a) et (2b) du collier, dans un plan perpendiculaire aux génératrices de la barre, pour être positionnée au dessus d'une face inclinée (4b) de la clavette (4). Un organe de serrage (5) vissé dans un talon (4c) de la clavette (4), prend appui sur la broche (3), pour assurer, lors de son serrage, le déplacement concomittant de la clavette, à l'encontre de ladite broche. La face inclinée (4b), sous l'effet du couple de serrage, créé une forte pression sur la barre de carbone (1).

Le système est mis en place dans un ordre précis, à savoir :
- mise en place du collier (2) sur la barre (1) à l'endroit voulu,
- positionnement de la clavette (4) entre les branches (2a) et (2b) du collier, l'organe (5) étant desserré,
- mise en place de la broche (3) au dessus de la face inclinée (4b) de la clavette (4),
- serrage de l'organe (5),

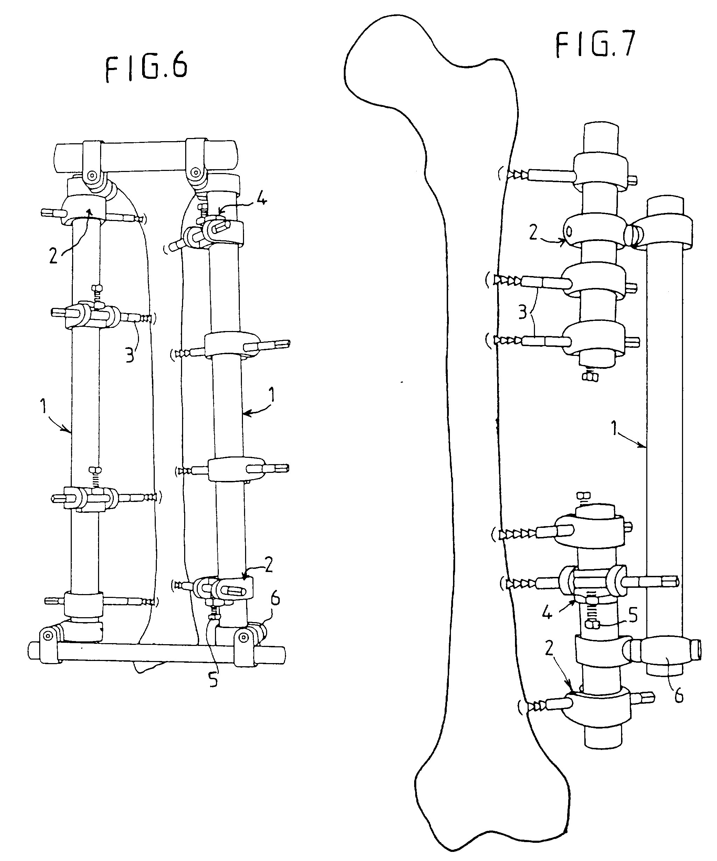

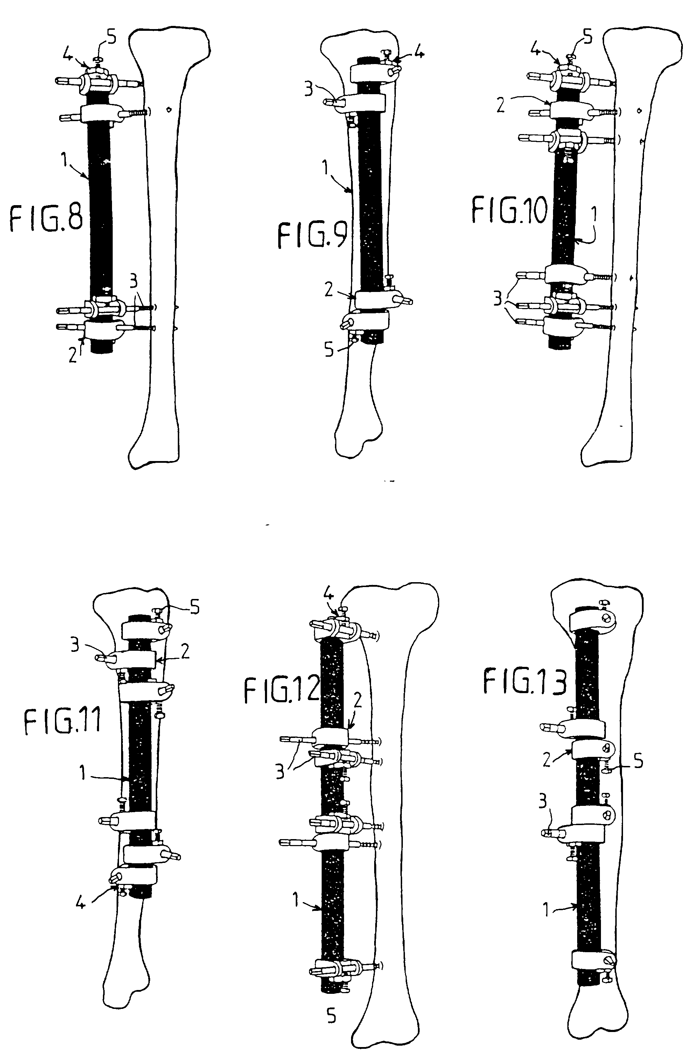

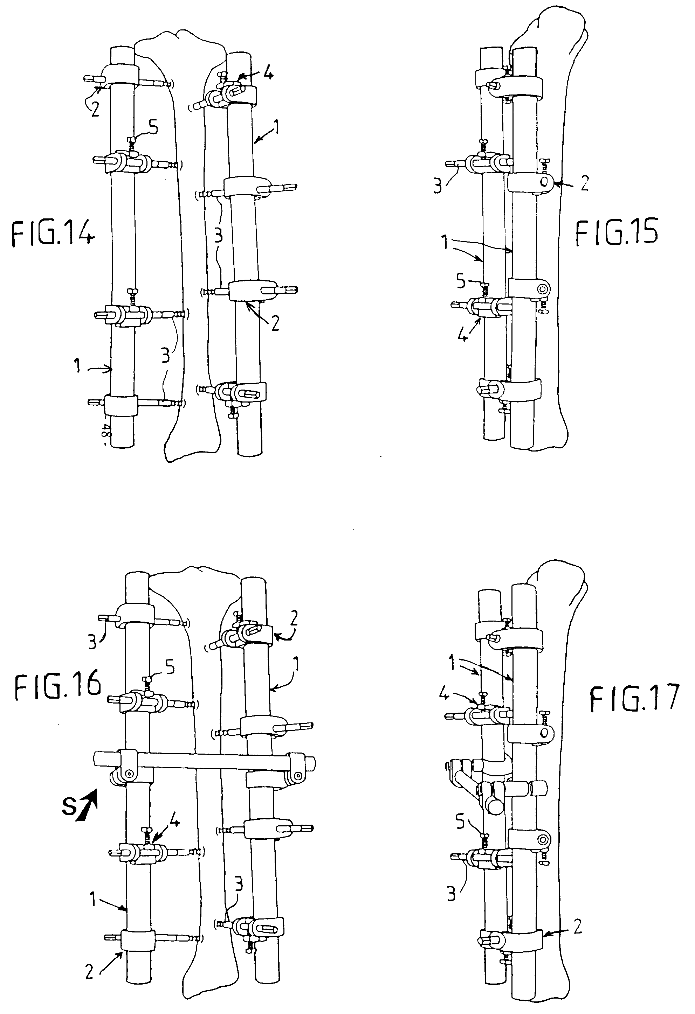

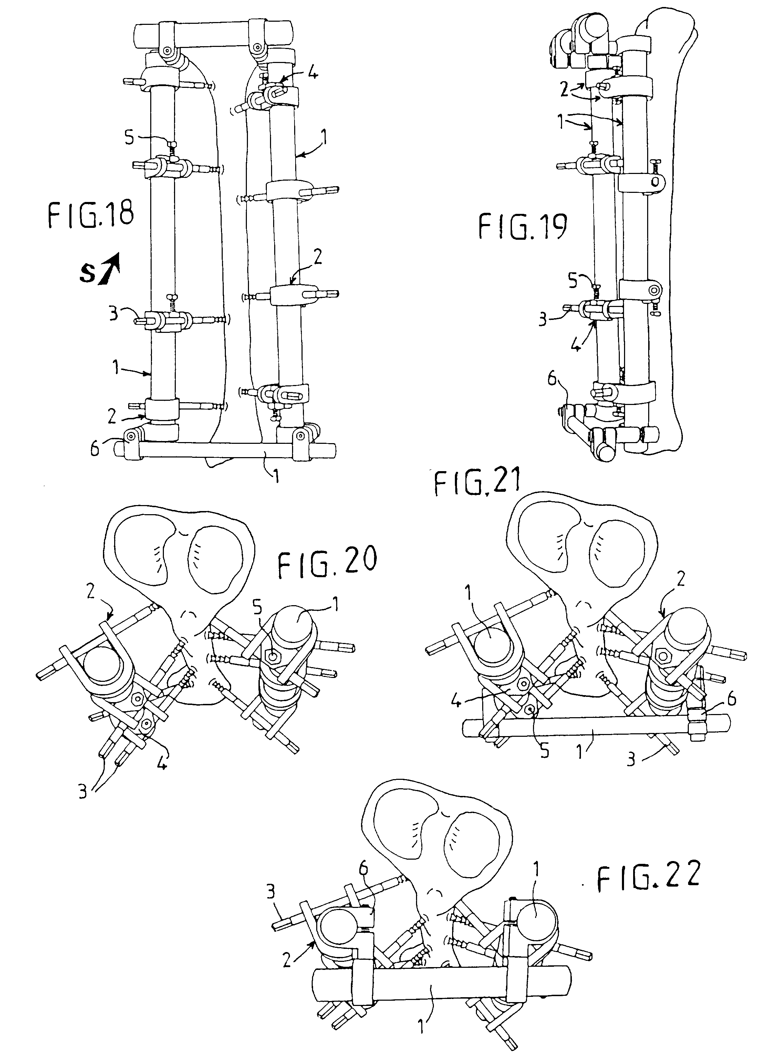

Il apparait donc que ces dispositions permettent d'avoir un système de fixation de broches unitaires, de sorte que chaque broche (3) est fixée directement sur la barre de carbone (1), par un système qui lui est propre. Chaque broche (3) peut donc prendre une position différente de sa voisine, et n'a donc pas de position préétablie, contrairement à la plupart des fixateurs connus de l'état de la technique. On renvoie aux figures 8 à 13, qui montrent un exemple de montage dans le cas d'un montage monobarre. A noter également, que la barre (1) peut recevoir des colliers doubles (6), pour permettre le montage d'autres barres (1) dans un plan parallèle ou perpendiculaire. On renvoie aux figures 6, 7, 16, 17, 18,19, 21 et 22.

Suivant une autre caractéristique, les extrémités des barres (1) peuvent coopérer avec des éléments d'accouplement susceptibles de recevoir, eux-mêmes, d'autres organes.

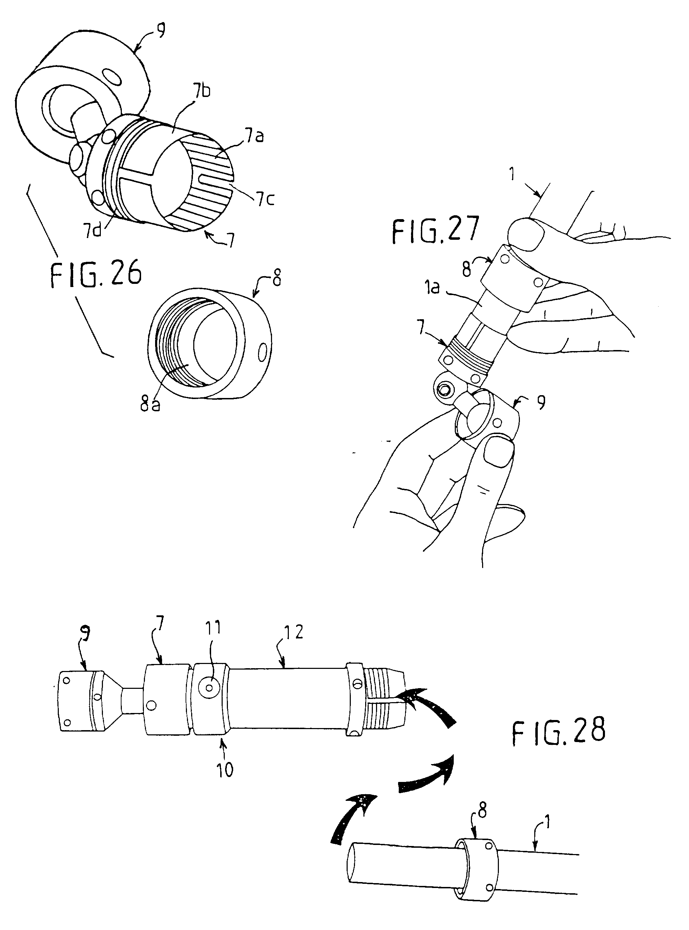

Comme le montrent les figures 26 et 27 notamment, ces éléments d'accouplement sont constitués par un embout (7), délimitant un chambrage interne (7a), apte à recevoir l'extrémité (la) d'une barre (1). Cet embout présente, à son extrémité, une portée conique mâle (7b), avec capacité de déformation élastique, en combinaison avec des fentes radiales (7c). A l'opposé de la portée conique (7b), l'embout (7) présente une portée filetée (7d), apte à recevoir une douille taraudée (8), présentant une portée interne tronconique (8a) complémentaire à la portée (7d). L'extrémité (1a) de la barre (1) est engagée dans le chambrage interne (7a), tandis que la douille (8), préalablement engagée sur la barre (1), est vissée sur la portée (7d), de sorte que sa portée tronconique (8a) déforme la portée mâle (7b), ce qui a pour effet d'assurer le serrage concomitant de la barre.

A noter que le chambrage interne (7a) peut être rainuré pour augmenter l'accrochage sur la barre (1).

Suivant une autre caractéristique, les extrémités des barres (1) peuvent également coopérer avec des éléments d'articulation complémentaires (9) et (10). L'élément (9) comprend un embout (9a) apte à recevoir la barre, au moyen par exemple, d'une vis. L'extrémité de l'embout (9a) présente une rotule sphérique (9b) montée avec capacité d'orientation angulaire, dans un siège (10a), formé par une douille (10b), conformée pour être vissée sur un embout (10c) de l'élément (10). Cet embout (10c) reçoit intérieurement une tête d'appui (10d), assujettie à une clavette (10e) présentant une pente pour faire plaquer ladite tête (10d) contre la rotule (9b), en position vissée de la douille (10b).

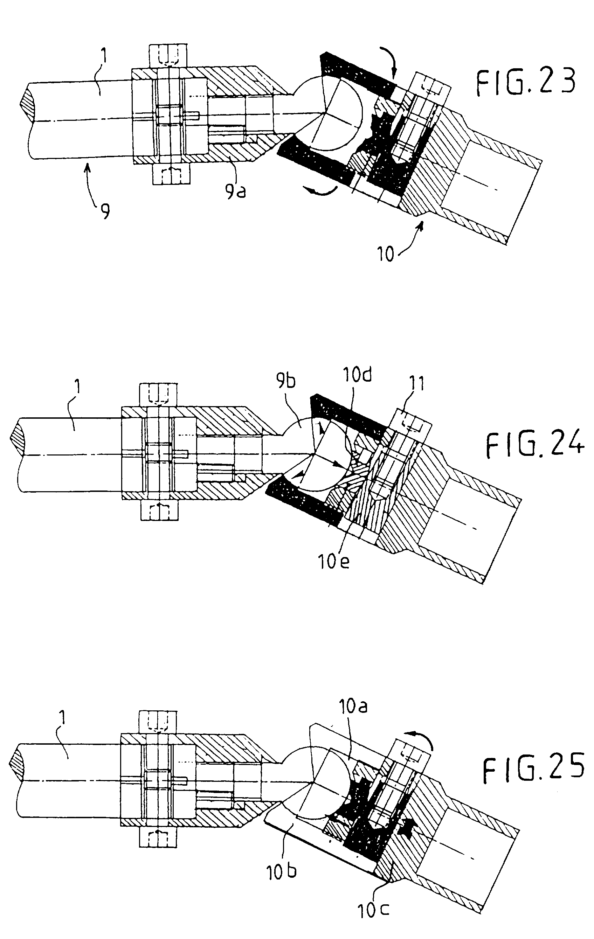

Une vis (11), qui coopère avec la clavette (10e), assure le blocage dans la position angulaire souhaitée de l'élément (9). On renvoie aux figures 23, 24 et 25.

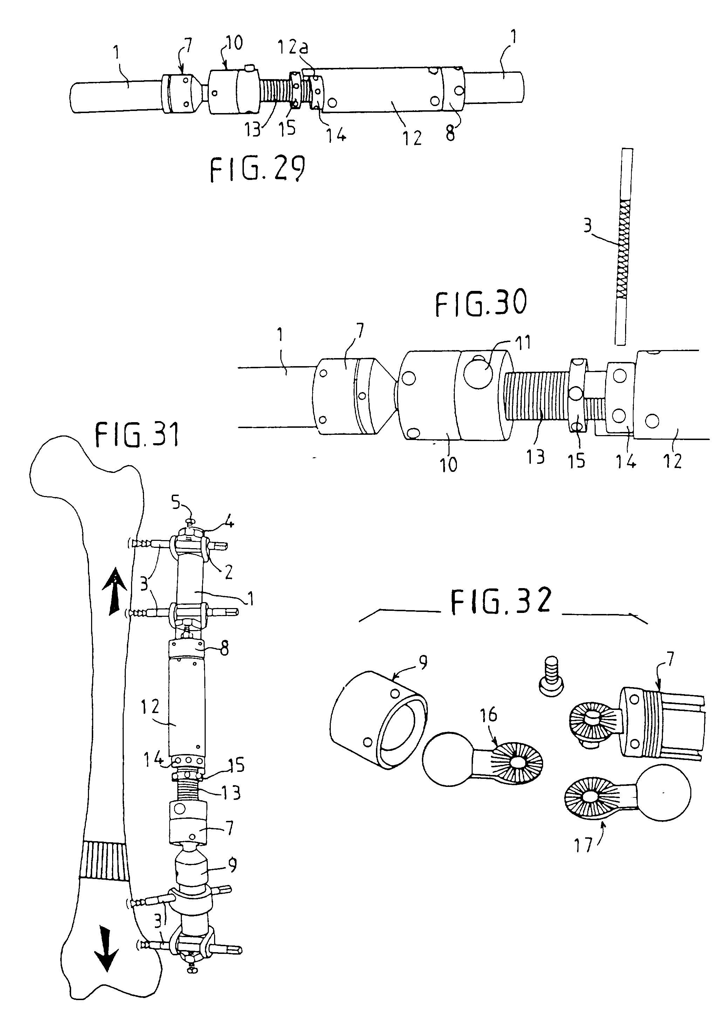

A noter que les éléments d'accouplement et d'articulation tels que décrits, peuvent coopérer directement ou de manière rapportée avec un corps (12). De même, l'une des extrémités de ce corps peut présenter un agencement identique à celui des éléments d'accouplement (7) (8), pour le montage d'une barre (figure 28). Le corps (12) peut également être équipé d'une tige d'allongement (13). Cette tige d'allongement (13) est vissée dans le corps, en étant déplacée en translation, par un écrou (14) logé dans un agencement du corps (12a), pour être bloqué en translation. Un contre-écrou (15) assure le blocage en position (figures 29 et 30).

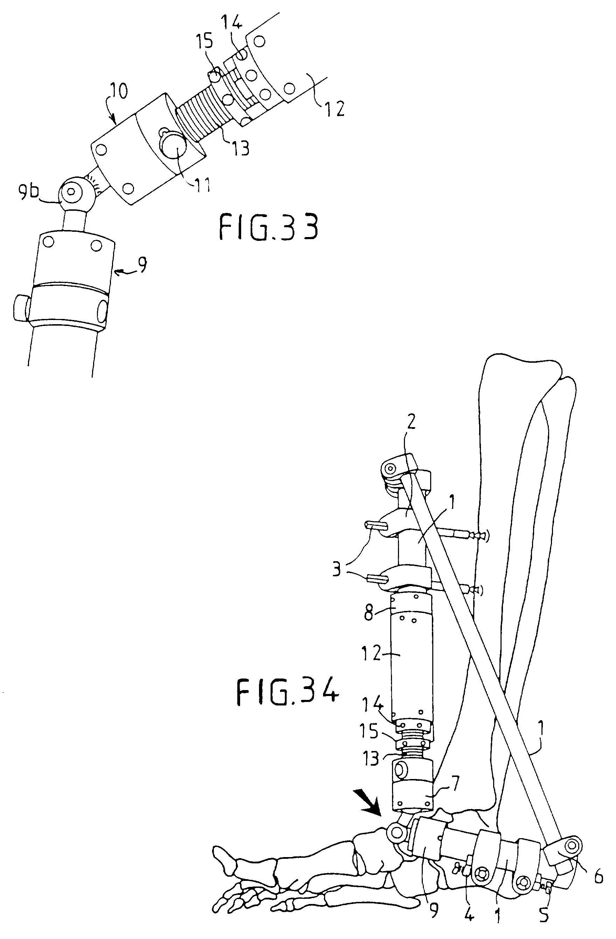

On prévoit également, comme le montre la figure 32, d'équiper les embouts (7) notamment, de renvois d'angles composés de deux demi-sphères crantées (16) (17) avec moyen de blocage en position. Ces dispositions permettent d'obtenir une forte angulation à un montage quelconque (figure 33).

L'une des sphères (16) peut être solidaire directement de l'embout (7), tandis que l'autre sphère (17) peut présenter, en bout d'une tige (17a), une rotule (17b) apte à coopérer par exemple, avec la douille (10a) de l'élément (10).

On renvoie aux différentes figures des dessins, qui donnent quelques exemples nullement limitatifs d'applications et de montages du fixateur selon l'invention, dans le cas notamment de traumatologie d'urgence des fractures ouvertes ou fermées, de ligamentotaxis, d'orthopédie froide des corrections axiales et allongements.

Les avantages ressortent bien de la description, en particulier on souligne et on rappelle :
- l'utilisation de barres de carbone très résistantes, susceptibles d'être coupées à la longueur souhaitée, en per ou pré-opératoire, le carbone étant par ailleurs radiotransparent,
- la combinaison des barres de carbone avec de nombreuses jonctions possibles, pour s'adapter à chaque cas clinique,
- la possibilité de monter le fixateur pièce par pièce, sans aucun gabarit,
- la jonction du fixateur pour aboutir à un montage terminé, pouvant s'effectuer où on le désire,
- le blocage en position, du système de rotule par une clavette spécifique permettant, dans un premier temps, un serrage modéré au moyen d'une douille, de manière à obtenir le frottement nécessaire pour maintenir l'angulation souhaitée, avec la possibilité de la modifier manuellement si cette dernière n'est pas correcte, la clavette étant bloquée par une vis, lorsque l'angulation parfaite souhaitée est obtenue, correspondant à la réduction souhaitée;
- l'indépendance des broches les unes par rapport aux autres,
- les montages en V possibles des broches,
- les nombreuses possibilités de positionnement des broches,
- la possibilité d'effectuer tous les types de montages, généralement préconisés, à savoir :
   * montage par simple barre sur fractures préréduites,
   * montage par deux barres en V,
   * montage du type HOFFMANN,
   * montages monolatéraux stricts.
- le caractère modulable.

## Revendications

1. Fixateur externe applicable en orthopédie et en traumatologie, comprenant des barres (1) en matériau composite, chaque barre (1) recevant, avec capacité de réglage en translation et de blocage en position, des organes indépendants d'ancrage dans l'os, constitués par un collier (2) recevant une broche (3) conformée pour être vissée dans l'os, ladite broche (3) étant bloquée sur le collier en combinaison avec un moyen rapporté (4), caractérisé en ce que le collier (2) enserre la demi circonférence d'une barre (1) avec capacité de déplacement le long de la barre et présente deux branches (2a) et (2b) recevant la broche (3) bloquée par le moyen (4) constitué par une clavette apte à être inserrée entre les branches (2a) et (2b), en appui sur les génératrices de la barre (1), au moyen d'une face (4a) de forme complémentaire, ladite broche (3) traversant les branches du collier, dans un plan perpendiculaire aux génératrices de la barre, pour être positionnée au dessus d'une face inclinée (4b) de la clavette (4), tandis qu'un organe de serrage (5) assure le déplacement de ladite clavette, à l'encontre de la broche en vue de sa fixation.

2. Fixateur selon la revendication 1, caractérisé en ce que les extrémités des barres (1) sont aptes à être accouplées à différents types d'éléments (7) (8) (9) (10) conformés pour faire varier leur longueur et/ou orientation angulaire.

3. Fixateur selon la revendication 2, caractérisé. en ce que les éléments d'accouplement des barres (1) sont constitués par un embout (7) délimitant un chambrage interne (7a) apte à recevoir l'extrémité de la barre (1) et présentant, à son extrémité, une portée conique mâle (7b) avec capacité de déformation élastique, à l'opposé de la portée conique, l'embout (7) reçoit une douille taraudée (8) présentant une portée interne tronconique femelle (8a) complémentaire à la précédente, pour assurer le blocage de la barre sous l'effet de déformation de la portée conique mâle résultant du vissage de ladite douille.

4. Fixateur selon la revendication 3, caractérisé en ce que le chambrage interne (7a) est rainuré pour augmenter l'accrochage sur la barre (1).

5. Fixateur selon la revendication 2, caractérisé en ce que les éléments comprennent un embout (9) apte à recevoir la barre (1), ledit embout présentant une rotule sphérique (9b) dont le siège (10a) est une douille (10b) conformée pour être vissée sur un corps (10c) équipé intérieurement d'une tête d'appui (10d) assujettie à une clavette (10e) présentant une pente pour faire plaquer, ladite tête contre la rotule en position vissée de son siège et dans la partie angulaire souhaitée de l'embout.

6. Fixateur selon la revendication 2, caractérisé en ce que les éléments d'articulation et d'accouplement sont montés en bout d'un corps (12).

7. Fixateur selon la revendication 6, caractérisé en ce que le corps (12) présente une tige d'allongement filetée (13) vissée dans le corps et déplaçable par un écrou (14) bloqué en translation dans un agencement du corps, un contre écrou (15) assurant le blocage en translation de la tige.

8. Fixateur selon l'une quelconque des revendications 3 et 5, caractérisé en ce que les embouts sont équipés de renvois d'angle (16) (17) composés de deux demi sphères crantées avec moyen de blocage en position.

9. Fixateur selon la revendication 1, caractérisé en ce que les barres recoivent des colliers doubles (6).
